(19) Europäisches Patentamt
European Patent Office
Office européen des brevets

(11) Veröffentlichungsnummer : **0 134 016**
**B1**

(12) **EUROPÄISCHE PATENTSCHRIFT**

(45) Veröffentlichungstag der Patentschrift : **15.06.88**

(51) Int. Cl.⁴ : **C 07 C 50/36**, C 07 C 46/00

(21) Anmeldenummer : **84109378.4**

(22) Anmeldetag : **08.08.84**

# (54) Verfahren zur Herstellung von Anthracyclinonen.

(30) Priorität : **12.08.83 DE 3329185**

(43) Veröffentlichungstag der Anmeldung : **13.03.85 Patentblatt 85/11**

(45) Bekanntmachung des Hinweises auf die Patenterteilung : **15.06.88 Patentblatt 88/24**

(84) Benannte Vertragsstaaten :
**AT BE CH DE FR GB IT LI NL SE**

(56) Entgegenhaltungen :
**CHEMICAL ABSTRACTS, Band 100, Nr. 3, 16. Januar 1984, Seite 476, Spalte 1, Abstract Nr. 22480s, Columbus, Ohio, US; K. KROHN et al.: "Synthetic anthracyclinones. XXII. Rhodomycinones of type A with methyl, ethyl, and n-propyl side chains", & LIEBIGS ANN. CHEM. 1983(10), 1818-1838**
**CHEMICAL ABSTRACTS, Band 96, Nr. 13, 29. März 1982, Seite 677, Spalte 2, Abstract Nr. 103933t, Columbus, Ohio, US; K. KROHN et al.: "Synthetic anthracyclinones. XVIII. Synthesis of 13-deoxo-6-deoxydaunomycinone and beta1-citromycinone", & LIEBIGS ANN. CHEM. 1981(12), 2285-2297**
**TETRAHEDRON LETTERS, Band 23, Nr. 4, 1982, Oxford,GB; K. KROHN et al.: "Synthese der Citromycinone. Stereoselektive Öffnung eines Epoxids zum cis-Diol", Seiten 395-398**

(73) Patentinhaber : **HOECHST AKTIENGESELLSCHAFT**
**Postfach 80 03 20**
**D-6230 Frankfurt am Main 80 (DE)**

(72) Erfinder : **Krohn, Karsten, Prof. Dr.**
**Am Turmsberg 49**
**D-3300 Braunschweig (DE)**
Erfinder : **Behnke, Bert, Dr.**
**Klaus-Groth-Strasse 24**
**D-2070 Ahrensburg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 134 016 B1

Jouve, 18, rue St-Denis, 75001 Paris, France

**Beschreibung**

Die vorliegende Erfindung betrifft ein neues Verfahren zur Herstellung von Anthracyclinonen der Formel

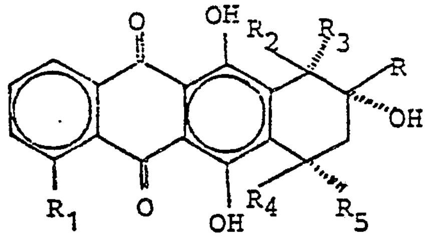

(I)

worin R $C_1$-$C_4$-Alkyl, $R_1$ H oder OH bedeuten, $R_2$ und $R_3$ verschieden sind und jeweils H oder OH darstellen und $R_4$ und $R_5$ verschieden sind und jeweils H oder OH darstellen.

Das Verfahren ist dadurch gekennzeichnet, daß man eine Verbindung der Formel

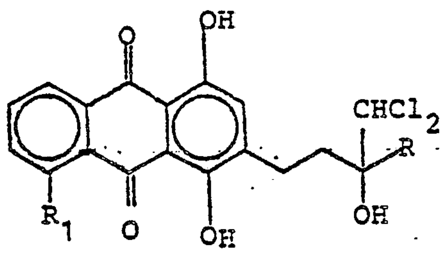

(II)

worin $R_1$ und R die oben genannten Bedeutungen haben, entweder

a) falls $R_1$ Wasserstoff bedeutet, mit einer Alkalilauge unter Zusatz eines Reduktionsmittels, vorzugsweise Natriumdithionit, bei 0 °C umsetzt und das erhaltene Produkt anschließend mit Luft oxidiert und ansäuert, wobei man eine Verbindung der Formel III

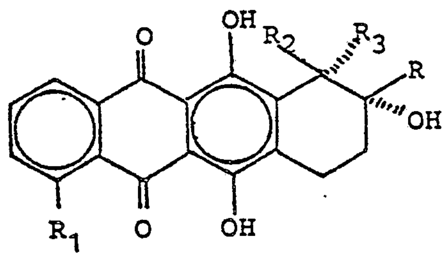

(III)

erhält, worin $R_1$ Wasserstoff bedeutet und R, $R_2$ und $R_3$ die oben angegebene Bedeutung haben, oder

b) falls $R_1$ die OH-Gruppe bedeutet, in einer Phasentransfer-Reaktion mit einer Alkalilauge in einem chlorierten Kohlenwassertoff, vorzugsweise Dichlormethan, unter Zusatz von Tetrabutylammoniumhydrogensulfat und anschließende Reduktion mit einem geeigneten Reduktionsmittel, vorzugsweise Natriumdithionit bei 0 °C umsetzt zu einer Verbindung der Formel III, worin $R_1$ die OH-Gruppe bedeutet und $R_1$, $R_2$ und $R_3$ die oben angegebene Bedeutung haben, oder

c) falls $R_1$ die OH-Gruppe und R $CH_3$ bedeutet mit Alkalimethanolat bei Raumtemperatur unter Inertgas umsetzt zu einer Verbindung der Formel IV

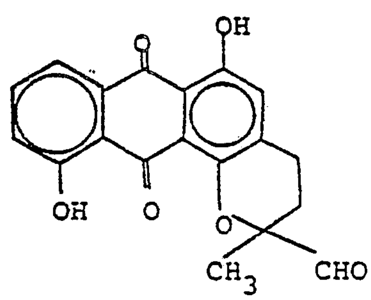

(IV)

und den erhaltenen cyclischen Aldehyd durch Umsetzen mit einem geeigneten Reduktionsmittel, vorzugsweise Natriumdithionit bei 0 °C und anschließende Luftoxidation und Ansäuren überführt in eine Verbindung der Formel III, worin R $CH_3$ bedeutet und $R_2$ und $R_3$ die oben angegebene Bedeutung haben, und

d) eine Verbindung der Formel III, worin R, $R_1$ und $R_3$ die oben genannte Bedeutung haben und $R_2$ die OH-Gruppe bedeutet, durch Veresterung mit Hexafluoressigsäureanhydrid in eine Verbindung der Formel III überführt, worin $R_2$ den Rest $—OCOCF_3$ darstellt, und

e) eine Verbindung der Formel III, worin R und $R_1$ die oben genannte Bedeutung haben, $R_3$ Wasserstoff und $R_2$ den Rest $—OCOCF_3$ darstellt, nach üblichen Methoden bromiert und anschließend hydrolysiert zu einer Verbindung der Formel I.

Die Verbindung der Formel II, worin $R_1$ Wasserstoff bedeutet, wird mit einer Alkalilauge, beispielsweise mit 1n Kalilauge bei ca. 25 °C zu dem labilen Aldehyd der Formel V

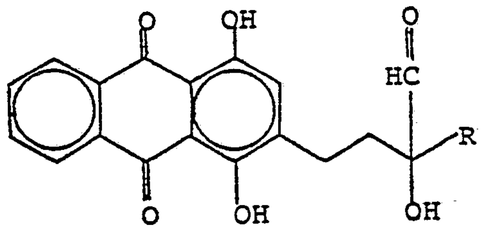

(V).

umgesetzt. Dabei wird die Verbindung der Formel II zweckmäßig mit Methanol angelöst, bevor unter Stickstoff die Lauge zugesetzt wird. Der nach ca. 10 min. praktisch quantitativ entstehende Aldehyd wird ohne Isolierung mit Natriumdithionit bei 0 °C zur Leukoform reduziert. Nach Luftoxidation und Ansäuern erhält man die cyclische Verbindung der Formel III.

Die Trennung der trans- und cis-Diole der Formel III kann über die leichtlösliche Acetonide der Formel VI

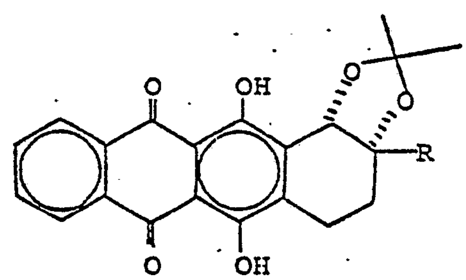

(VI)

erfolgen, die durch Behandlung des Rohprodukts der Cyclisierung mit Aceton/p-Toluolsulfonsäure quantitative hergestellt werden.

Falls in der Formel II $R_1$ die OH-Gruppe bedeutet, wird die Verseifung der Dichloride mittels einer Phasen-transfer-Reaktion durchgeführt. Dabei werden die α-Hydroxydichloride in einem System aus einem chlorierten Kohlenwasserstoff, vorzugsweise Dichlormethan und einer Alkalilauge, z. B. 0,2 n NaOH unter Zusatz von Tetrabutylammoniumhydrogensulfat innerhalb einer Stunde quantitativ zu den entsprechenden Aldehyden umgesetzt. Die labilen Aldehyde werden ohne Isolierung durch Reduktion mit einem geeigneten Reduktionsmittel, vorzugsweise Natriumdithionit bei 0 °C zu den tetracyclischen Verbindungen der Formel III umgesetzt. Man kann ein α-Hydroxydichlorid der Formel II, worin $R_1$ OH und R $CH_3$ bedeutet, auch durch Behandlung mit Alkalialkanolat, vorzugsweise Natriummethanolat bei Raumtemperatur unter Inertgas in den cyclischen Aldehyd der Formel IV überführen. Dieser ebenfalls labile Aldehyd läßt sich mit einem geeigneten Reduktionsmittel, vorzugsweise Natriumdithionit bei 0 °C und anschließende Luftoxidation und Ansäuern in hohen Ausbeuten in die tetracyclische Verbindung der Formel III überführen.

Die Einführung der OH-Gruppe in 10-Stellung gelingt durch die üblichen Methoden der radikalischen Bromierung. Dazu muß jedoch die trans-Hydroxygruppe and C—7 ($R_2$ = OH) durch Überführung in das Trifluoroacetat geschützt werden, was durch Veresterung mit Hexafluoressigsäureanhydrid geschieht. Das resultierende Trifluoracetat wird in einem chlorierten Kohlenwasserstoff, z. B. Tetrachlorkohlenstoff, unter Lichteinwirkung 1-5 Stunden, vorzugsweise 2-3 Stunden bei Raumtemperatur bromiert. Das entstehende Gemisch der labilen Bromide wird ohne Isolierung in einem Zweiphasensystem aus Ether und eiskalter Alkalilauge, z. B. 1n Natronlauge umgesetzt. Nach dem Ansäuern erhält man das 8,10-Diol der Formel I.

Die Ausgangs- bzw. Zwischenprodukte der Formel II können auf folgende Weise hergestellt werden :

An eine Verbindung der Formel VII

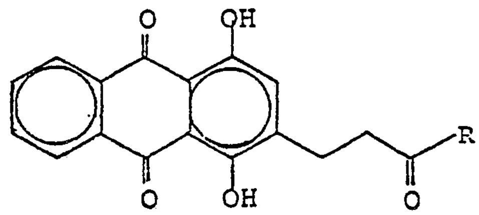

(VII)

worin R $C_1$-$C_4$-Alkyl darstellt wird Lithiumdichlormethan bei Temperaturen von —90 bis —100 °C addiert. Die Reaktion wird so ausgeführt, daß das Dichlormethan bei ca. —100 °C in Tetrahydrofuran mit n-Butyllithium metalliert wird und das Keton der Formel VII bei dieser Temperatur mit dem Carbenoid umgesetzt wird. Die anschließende Neutralisierung des Reaktionsgemisches wird ebenfalls bei —90 °C bis —100 °C durchgeführt. Die Verbindungen der Formel II werden dabei mit Ausbeuten zwischen 90 und 95 % erhalten.

Die bisher bekannten Anthracyclinon-Synthesen beruhen entweder auf der Diels-Alder-Reaktion (A.S. Kende, Y. Tsay, J. Chem. Soc., Chem. Commun. 1977, 140 ; K. Krohn, A. Rösner, Liebigs Ann. Chem. 1979, 2018) oder einer intramolekularen Variante der Marschalk-Reaktion (C. Marschalk, F. Krenig, N. Ouronsoff, Bull. Soc. Chim. Fr. 3 (1936), 1545). Bei diesen Reaktionen sind jedoch mehrere Stufen notwendig, z. B. ist eine dreistufige Reaktionsfolge zur Spaltung der α-Hydroxydithioacetale erforderlich. Ein weiterer Nachteil besteht darin, daß die Ausbeuten bei der Herstellung der entsprechenden α-Hydroxyaldehyde mittels lithiiertem 1,3-Dithian durch eine teilweise Reduktion zu den Leuko-Anthrachinonen herabgesetzt werden. Demgegenüber führt das erfindungsgemäße Verfahren in einer Eintopfreaktion von den Verbindungen der Formel II zu den Anthracyclinonen der Formel III bei Ausbeuten von 80 %.

Die Verbindungen der Formel I lassen sich auf üblichem Wege durch Substitution in 7- und/oder 10-Stellung in die entsprechenden Glykoside mit cytostatischer (antitumoraler) Wirksamkeit umwandeln (s. z. B. US Patent Nr. 3 988 315, 4 071 411 entspr. DE Patentschrift 2 532 568).

## Beispiele

Schmelzpunkte (unkorrigiert) : Apparat der Fa. Elektrothermal. — IR-Spektren : Perkin-Elmer-Gerät 297. — UV-Spektren (in Methanol) : Zeiss Spektralphotometer DMR 10. — $^1$H-NMR-Spektren (Tetramethylsilan als innerer Standard) : Bruker WH 270 (270 MHz) und WH 400 (400 MHz) oder Perkin-Elmer R 32 (90 MHz) ; Lösungsmittel $CDCl_3$). — MS : m/e = Varian-MAT-Geräte CH 7 (70 eV) ; RDA bezeichnet Retro-Diels-Alder-Fragment.

Allgemeine Arbeitsvorschrift für die Synthese der α-Hydroxydichloride (II)

Eine Mischung aus 150 ml Tetrahydrofuran und 3,06 g (36 mmol) Dichlormethan wurde bei —100°C mit 23,3 ml 1.6 N Butyllithium innerhalb von 30 min versetzt. Nach weiteren 30 min bei —100 °C wurde eine Lösung des entsprechenden Ketons (VII) über einen Zeitraum von 1-2 h zugegeben ; die Temperatur durfte dabei —95 °C nicht übersteigen. Nach 1 h bei —100 °C wurde die Lösung vorsichtig mit 6 N Salzsäure neutralisiert und nach Erwärmung auf Raumtemperatur mit 200 ml Dichlormethan versetzt. Die dreimal mit Wasser gewaschene und über Natriumsulfat getrocknete Lösung wurde i. Vak. eingeengt und der Rückstand aus wenig Dichlormethan kristallisiert.

### Beispiel 1

1,4-Dihydroxy-2-(4,4-dichlor-3-hydroxy-3-methylbutyl)-9,10-anthrachinon (II)

Aus 1,59 g (5.1 mmol) Keton (VII, R = $CH_3$) erhielt man 1,90 g (95 %) mit Schmp. 148 °C — IR : 3440 (OH), 1620 (Chinon cheliert), 1585 (C=C). — UV : λ max (1 g ε) = 226 (4.39), 246 (4.65), 279 (4.10), 318 (3.71), 456 (4.04), 478 (4.07), 508 nm (3.90). — $^1$H-NMR : = 1.48 (s ; 3H, $CH_3$), 2.09 (mc ; 2H, 2'—$CH_2$), 2.32 (s ; 1H, OH), 2.89 (mc ; 2H, 1'—$CH_2$), 5.74 (s ; 1H, $CHCl_2$), 7.16 (s ; 1H, 3—H), 7.80 (mc ; 6—, 7—H), 8.31 (mc ; 2H, 5—, 8—H), 12.90, 13.39 (je s ; je 1H, je OH).

$C_{19}H_{16}O_5Cl_2$ (395.2)
Ber. : C 57.74 H 4.07 Cl 17.94
Gef. : C 57.81 H 4.02 Cl 18.18

### Beispiel 2

1,4-Dihydroxy-2-(3-dichlormethyl-3-hydroxypentyl)-9,10-anthrachinon (II)

Aus 570 mg (1.76 mmol) Keton (VII) R = $C_2H_5$ erhielt man 655 mg (91 %) mit Schmp. 119 °C. — IR : 3450 (OH), 1620 (Chinon cheliert), 1585 (C=C), Bereich 800-720 $cm^{-1}$ (C—Cl). — UV. $\lambda_{max}$ (1 g ε) = 225

(4.27), 246 (4.51), 281 (3.97), 317 (3.43), 456 (3.93), 477 (3.97), 510 nm (3.77). — $^1$H-NMR : = 1.04 (t ; J = 7.6 Hz ; 3H, $CH_3$), 1.93 (q ; J = 7.6 Hz ; 2H, $CH_2CH_3$), 2.10 (mc ; 2H, 2'—$CH_2$), 2.22 (s ; 1H, OH), 2.85 (mc ; 2H, 1'—$CH_2$), 5.91 (s ; 1H, $CHCl_2$), 7.19 (s ; 1H, 3—H), 7.83 (mc ; 2H, 6—, 7—H), 8.34 (mc ; 2H, 5—, 8—H), 12.94, 13.42 (je s ; je 1H, je OH).

$C_{20}H_{18}O_5Cl_2$ (409.3)
Ber. : C 58.70 H 4.43 Cl 17.32
Gef. : C 58.99 H 4.47 Cl 17.39

Beispiel 3

3-(4,4-Dichlor-3-hydroxy-3-methylbutyl)-1,4,5-trihydroxy-9,10-anthrachinon (II)

Aus 650 mg Keton (VII) R = $CH_3$ (s. unten) erhielt man 771 mg (94 %) mit Schmp. 201 °C (Zers.). — IR : 3450 (OH), 2960, 2925 (CH), 1600 (Chinon cheliert, C=C). — UV : $\lambda_{max}$ (1 g ε) = 229 (4.54), 245 (4.23), 285 (3.81), 460 (3.94), 487 (4.05), 508 (3.93), 520 nm (3.86. — $^1$H-NMR : = 1.55 (s ; 3H, $CH_3$), 2.09 (mc ; 2H, 2'—$H_2$), 2.19 (s ; 1H, OH), 2.90 (mc ; 2H, 1'—$H_2$), 5.79 (s ; 1H, CH—$Cl_2$), 7.22 (s ; 1H, 2—H), 7.31 (dd, $J_{6,7}$ = 8.2, $J_{6,8}$ = 1.2 Hz ; 1H, 6—H), 7.71 (dd, $J_{6,7}$ = 8.2, $J_{7,8}$ = 7.6 Hz ; 1H, 7—H), 7.89 (dd, $J_{7,8}$ = 7.6, $J_{6,8}$ = 1.2 Hz ; 1H, 8—H), 12.23, 12.81, 13.09 (je s ; je 1H, je OH).

$C_{19}H_{16}O_6Cl_2$ (411.2)
Ber. : C 55.50 H 3.92 Cl 17.24
Gef. : C 55.24 H 3.87 Cl 17.13

Beispiel 4

3-(3-Dichlormethyl-3-hydroxypentyl)-1,4,5-trihydroxy-9,10-anthrachinon (II)

Aus 700 mg Keton (VII, R = $C_2H_5$) erhielt man 805 mg (92 %) mit Schmp. 174 °C. — IR : 3475 (OH), 2960, 2930, 2880 (CH), 1600 (Chinon cheliert, C=C). — UV : $\lambda_{max}$ (1 g ε) = 288 (4.58), 245 (4.34), 284 (3.97), 461 (3.99), 487 (4.07), 510 (3.96), 520 nm (3.91). — $^1$H-NMR : 1.04 (t, J = 7.4 Hz ; 3H, $CH_3$), 1.93 (q, J = 7.4 Hz ; 2H, $CH_2CH_3$), 2.09 (mc ; 2H, 2'$H_2$), 2.15 (s ; 1H, OH), 2.85 (mc ; 2H, 1'—$H_2$); 5.91 (s ; 1H, CH—$Cl_2$), 7.22 (d, J = 0.5 Hz ; 1H, 2—H), 7.31 (dd, $J_{6,7}$ = 8.4, $J_{6,8}$ = 1.2 Hz ; 1H, 6—H), 7.71 (dd, $J_{6,7}$ = 8.4, $J_{7,8}$ = 7.6 Hz, 7—H), 7.89 (dd, $J_{7,8}$ = 7.6, $J_{6,8}$ = 1.2 Hz ; 1H, 8—H), 12.22, 12.80, 13.09 (je s ; 1H, je OH).

$C_{20}H_{18}O_6Cl_2$ (425.3)
Ber. : C 56.49 H 4.27 Cl 16.67
Gef. : C 56.28 H 4.21 Cl 16.63

Beispiel 5

3-(3-Dichlormethyl-3-hydroxyhexyl)-1,4,5-trihydroxy-9,10-anthrachinon (II)

Aus 1.00 g Keton (VII, R = n—$C_3H_7$) erhielt man 1.10 g (89 %) mit Schmp. 175 °C (Zers.). — IR. 3475 (OH), 2960, 2920, 2870 (CH), 1600 (Chinon cheliert, C=C). — UV : $\lambda_{max}$ (1 g ε) = 232 (4.57), 250 (4.33), 290 (3.89), 434 (3.72), 462 (4.01), 479 (4.07), 489 (4.11), 511 (3.99); 523 nm (3.95). — $^1$H-NMR : = 1.01 (t, J = 7.2 Hz ; 3H, $CH_3$), 1.49 (mc ; 2H, $CH_2CH_2CH_3$), 1.84 (mc ; 2H, $CH_2CH_2CH_3$), 2.09 (mc ; 2H, 2'—$H_2$), 2.16 (s ; 1H, OH), 2,85 (mc ; 2H, 1'—$H_2$), 5.88 (s ; 1H, CH—$Cl_2$), 7.22 (s ; 1H, 2—H), 7.31 (dd, $J_{6,7}$ = 8.4, $J_{6,8}$ = 1,2 Hz ; 1H, 6—H), 7.71 (t; 1H, 7—H), 7.89 (dd, $J_{7,8}$ = 7.6, $J_{6,8}$ = 1.2 Hz, 1H, 8—H), 12.21, 12,79, 13.07 (je s ; je 1H, je OH) :

$C_{21}H_{20}O_6Cl_2$ (439.3)
Ber. : C 57.42 H 4.59 Cl 16.14
Gef. : C 57.31 H 4.52 Cl 16.03

Beispiel 6

(+) (-1-Desoxy-$\beta_1$-rhodomycinon (III, R = $CH_3$, $R_2$ = OH, $R_3$ = H)

Eine Lösung von 1.9 g (4.81 mmol) Dichlorid (II, R = $CH_3$) in 40 ml Methanol wurde bei Raumtemperatur unter Stickstoff mit 100 ml 1N Kalilauge versetzt und 10 min gerührt. Dann kühlte man auf 0 °C ab, setzte so lange Natriumdithionit-Lösung (ca. 5 mmol) zu, bis die Farbe von Blau nach Orange umschlug und rührte 20 min bei 0 °C. Zur Oxidation wurde Luft in die Lösung eingeleitet und stark gerührt. Durch Ansäuern mit kalter 6N Salzsäure wurde das Produktgemisch ausgefällt. Man extrahierte zweimal mit je 300 ml Dichlormethan, wusch die Lösung mit wasser, trocknete über Natriumsulfat, filtrierte und dampfte das Lösungsmittel ab. Das Rohprodukt (cis/trans-Gemisch : Ausbeute 1.4 g) wurde in 100 ml Aceton aufgenommen, mit 30 mg p-Toluolsulfonsäure versetzt und fünf Stunden bei Raumtemperatur stehen gelassen. Dann wurden 300 ml Wasser zugesetzt und mit 150 ml Dichlormethan extrahiert. Die organische Phase wusch man zweimal mit Wasser, trocknete über Natriumsulfat, filtrierte und engte im Vakuum bis auf 8 ml ein. Es kristallisierten (12 h, 4 °C) 1.0 g (61 %) des schwerlöslichen trans-Diols (= Titel-Verb.) mit Schmp. 246 °C (Zers.). Das Rhodomycinon war identisch mit einer früher

bereiteten Probe (K. Krohn, B. Behnke, Liebigs Ann. Chem. 1979, 2011).

Beispiel 7

Isopropylidenether (VI, R = $CH_3$)

In der Mutterlauge (s. oben) lag der Isopropylidenether de cis-Diols (III, R = $CH_3$, $R_2$ = H, $R_3$ = OH) vor, der durch Zusatz von wenig Petrolether kristallisierte und identisch war mit einer Referenzprobe. Ausbeute 452 mg (25 %) ; Schmp. 152 °C.

Beispiel 8

(+)-1-Desoxy-7-epi-$\beta_1$-rhodomycinon (III, R = $CH_3$, $R_2$ = H, $R_3$ = OH)

Eine Lösung von 3.8 mg (0.1 mmol) des Acetonids (Beispiel 7) in Tetrahydrofuran/Methanol wurde mit 0.2 ml konz. Salzsäure versetzt und nach 4 h zur Trockne eingedampft. Ausbeute quantitativ ; Schmp. 203 °C (Zers.).

Beispiel 9

(+)-1-Desoxy-$\gamma$-rhodomycinon (III, R = $C_2H_5$, $R_2$ = OH, $R_3$ = H)

250 mg (0.61 mmol) Dichlorid (II, R = $C_2H_5$) wurden wie in Beispiel 6 beschrieben umgesetzt. Ausbeute 136 mg (63 %) ; Schmp. 214 °C. — IR : 3520 (OH), 1610 (Chinon cheliert), 1580 (C=C). — UV : $\lambda_{max}$ (1 g ε) = 249 (4.45), 284 (3.76), 456 (3.79), 480 (3.86), 514 nm (3.70). — $^1$H-NMR : (Pyridin-$d_5$, TMS=0) δ = 1.38 (t ; J = 7.6 Hz ; 3H, $CH_3$), 2.13 (mc ; 2H, $CH_2CH_3$, 9a—H), 2.32 (mc ; 1H, $CH_2CH_3$), 2.41 (dddd ; $J_{gem}$ = 13.8, $J_{9e,10a}$ = 6.4, $J_{9e,10e}$ = 3.6, $J_{9e,7e}$ = 1.2 Hz ; 1H, 9e—H), 3.25 (mc ; 2H, 10a, e—H), Bereich 4.92-6.09 (breites Signal ; 2H, 20H), 5.49 (d ; $J_{9e,7e}$ = 1.2 Hz ; 1H, 7—H), 7.71 (mc ; 2H, 2—, 3—H), 8.36 (mc ; 2H, 1—, 4—H), 13.83, 14.17 (je s ; je 1H, je OH). — MS (195 °C) : m/e = 354 ($M^+$, 53 %), 336 (30), 307 (15), 284 (11), 282 (RDA, 33), 280 (24), 279 (44), 255 (18), 254 (100), 239 (16).

$C_{20}H_{18}O_6$ (354,4)
Ber. : C 67.79 H 4.56
Gef. : C 67.48 H 4.60

Beispiel 10

cis-3a-Ethyl-6,13-dihydroxy-2,2-dimethyl-3a,4,5,7,12,13b-hexahydronaphthaceno-[1,2-d]-1,3-dioxol-7,12-dion (VI, R = $C_2H_5$)

Aus der Mutterlauge (s. oben) kristallisierten nach Zugabe von wenig Petrolether 55 mg (23 %) des Acetonids mit Schmp. 300 °C. — IR : 1620 (Chinon cheliert), 1580 (C=C). — UV : $\lambda_{max}$(lgε) = 230 (4.19), 234 (4.21) 251 (4.17), 285 (3.65), 292 (3. 65), 317 (3.33), 472 (3.49), 482 (3.51), 506 nm (3.37). — $^1$H-NMR : δ = 1.01 (t ; J = 7.4 Hz ; 3H, $CH_2CH_3$), 1.32 (s ; 3H, $CH_3$), 1.53 (s ; 3H, $CH_3$), 1.64 (mc ; 2H, $CH_2CH_3$), 1.71 (dt ; $J_{gem}$ = 14.2, J = 4.8 Hz ; 1H, 9—H), 2.14 (ddd, $J_{gem}$ = 14.2, J = 6.4, J = 5.5 Hz ; 1H, 9—H), 2.90 (mc ; 2H, 10—H), 5.17 (s ; 1H, 7a—H), 7.83 (mc ; 2H, 2—, 3—H), 8.36 (mc ; 2H, 1—, 4—H), 13.38, 13.64 (je s ; je 1H, je OH). — MS (255 °C) : m/e = 395 (M + 1,17 %), 394 ($M^+$, 67 %), 379 (36), 338 (17), 337 (78), 336 (56), 321 (12), 320 (15), 319 (43), 318 (18), 309 (21), 308 (100), 307 (60), 293 (12), 291 (16), 290 (14), 281 (17), 280 (52), 279 (28) 187 (35).

Beispiel 11

(+)-1-Desoxy-7-epi-$\gamma$-rhodomycinon (III, R = $C_2H_5$, $R_2$ = H, $R_3$ = OH)

Eine Lösung von 50 mg Acetonid gemäß Beispiel 10 (0.13 mmol) in 10 ml Tetrahydrofuran wurde bei Raumtemperatur mit 2 ml 6N Salzsäure versetzt und 12 h gerührt. Man verdünnte mit Wasser, extrahierte mit Dichlormethan, trocknete die organische Phase über Natriumsulfat, filtrierte und engte i.Vak. ein. Aus Dichlormethan kristallisierten 44 mg (99 %) cis-Diol mit Schmp. 239 °C. — IR : 3400 (OH), 1620 (Chinon cheliert), 1580 (C=C). — UV : $\lambda_{max}$(1gε) = 248 (4.46), 285 (3.84), 457 (3.83), 477 (3.90) 509 (3.71), 562 (2.93). — $^1$H-NMR : δ = 1.03 (t ; J = 7.4 Hz ; 3H, $CH_2CH_3$), 1.62 (dq ; $J_{gem}$ = 21.0, J = 7.4 Hz ; 2H, $CH_2CH_3$), 1.76 (dt ; $J_{gem}$ =6.6 Hz ; 1H, 9—H), 2.10 (ddd, $J_{gem}$ = 13.4, J = 7.0, J = 6.0 Hz ; 1H, 9—H), 2.76 (ddd, $J_{gem}$ = 19.2, J = 7.4, J = 6.0 Hz ; 1H, 10—H), 2.80 (s ; 1H, OH), 3.05 (dt, $J_{gem}$ = 19.2, J = 7.2 Hz ; 1H, 10—H), 3.82 (s breit ; 1H, OH), 4,82 (s breit ; 1H, 7—H), 7.85 (mc ; 2H, 2—, 3—H), 8.36 (mc ; 2H, 1—, 4—H), 13.35, 13.90 (je s ; je 1H, je OH). — MS (210 °C) : m/e = 354 ($M^+$, 15 %), 336 (7), 282 (RDA, 9), 279 (12), 254 (26).

## Beispiel 12

(+)-1,7-Didesoxy-γ-rhodomycinon-7yl-trifluoracetat (III, $R = C_2H_5$, $R_2 = OCOCF_3$, $R_3 = H$)

Eine Lösung von 50 mg (0,14 mmol) (±)-1-Desoxy-$\beta_1$-rhodomycinon (Beispiel 6) in 3 ml Hexafluoressigsäureanhydrid wurde nach 5 h bei 20 °C i.Vak. zur Trockene eingedampft und der Rückstand aus Dichlormethan/Petrolether kristallisiert. Ausbeute 61 mg (98 %) ; Schmp. 164 °C. — IR : 3500 (OH), 1790 (C=O, Trifluoracetat), 1700-1730 (breite C=O-Schwingung für ein uncheliertes Chinon), 1620 (Chinon cheliert), 1580 (C=C). — UV : $\lambda_{max}$(1gε) = 247 (4.46), 282 (3.84), 456 (3.85), 480 (3.93), 512 (3.77), 553 nm (3.05). — $^1$H-NMR : δ = 1.10 (t ; J = 7.5 Hz ; 3H, $CH_2CH_3$), 1.70 (dq, $J_{gem}$ = 24.9, J = 7.5 Hz ; 2H, $CH_2CH_3$), 1.85 (ddd, $J_{9a,9e}$ = 14.2, $J_{9a,10a}$ = 11.6, $J_{9a,10e}$ = 6.2 Hz ; 1H, 9a—H), 2.11 (dddd, $J_{gem}$ = 14.2, $J_{9e,10a}$ = 6.4, $J_{9e,10e}$ = 4.4, $J_{9e,7e}$ = 1.2 Hz ; 1H, 9—H), 289 (ddd, $J_{gem}$ = 19.6, $J_{9a,10a}$ = 11.6, $J_{10a,9e}$ = 6.4 Hz ; 1H, 10—H), 3.12 (ddd, $J_{gem}$ = 19.6, $J_{10e,9a}$ = 6.2, $J_{10e,9e}$ = 4.4 Hz ; 1H, 10—H), 6.26 (d, $J_{9e,7e}$ = 1.2 Hz ; 1H, 7—H), 7.83 (mc ; 2H, 2—, 3—H), 8.33 (mc ; 2H, 1—, 4—H), 13.31, 13.45 (je s ; je 1H, je OH). — MS (300 °C) : m/e = 450 ($M^+$, 35 %), 338 (9), 337 (14), 336 (47), 320 (14), 318 (23), 309 (22), 308 (100), 307 (29), 293 (15), 291 (13), 282 (11), 281 (34), 280 (24), 279 (45), 254 (19).

## Beispiel 13

(+)-1-Desoxy-$\alpha_1$-rhodomycinon (I, $R = CH_3$, $R_2 = OH$, $R_4 = H$, $R_5 = OH$)

Eine Lösung von 100 mg (0.23 mmol) Trifluoracetat (III, $R = CH_3$, $R_2 = OCOCF_3$, $R_3 = H$) in 20 ml trockenem Tetrachlorkohlenstoff wurde bei Raumtemperatur mit einem Tropfen Brom versetzt und 2 h mit einer 100 W-Lampe bestrahlt. Nach 1 h wurde nochmals ein Tropfen Brom zugesetzt (DC-Kontrolle). Dann engte man die Lösung i.Vak. bei Raumtemperatur ein, suspendierte den Rückstand in 10 ml Ether, versetzte mit 5 ml kalter 1 %iger Natronlauge und rührte drei Minuten. Nach dem Ansäuern mit 6N Salzsäure wurde mit Dichlormethan extrahiert, über Natriumsulfat getrocknet und i.Vak. eingeengt. Den Rückstand trennte man schichtchromatographisch auf (Dichlormethan/Ether 96 : 4). Aus der polaren Hauptfraktion kristallisierten 36 mg (34 %) Triol mit Schmp. 143 °C (Zers.). — IR : 3400 (OH), 1620 (Chinon cheliert), 1580 (C=C). — UV : $\lambda_{max}$(1gε) = 223 (4.04), 245 (4.37), 278 (3.64), 319 (3.27), 448 (3.68), 478 (3.82), 513 nm (3.59). — $^1$H-NMR : δ = 1.53 (s ; 3H, $CH_3$), 2.19 (ddd, $J_{gem}$ = 15.2, $J_{9e,10e}$ = 2.0, $J_{9e,7e}$ = 1.2 Hz ; 1H, 9e—H), 2.26 (dd ; $J_{gem}$ = 15.2, $J_{9a,10e}$ = 4.6 Hz ; 1H, 9a—H), 2.76 (mc ; 1H, OH), 3.46 (mc ; 1H, OH), 3.51 (mc ; 1H, OH), 4.84 (dd, $J_{7e,7—OH}$) = 4.8, $J_{7e,9e}$ = 1.2 Hz ; 1H, 7e—H), 5.25 (mc ; 1H, 10e—H), 7.86 (mc ; 2H, 2—, 3—H), 8.37 (mc ; 2H, 1—, 4—H), 13.44, 13.51 (je s ; Je 1H, je OH). — MS (ungeheizt) : m/e = 356 ($M^+$, 3 %), 338 (16), 336 (20), 321 (20), 320 (100), 305 (13), 304 (58), 298 (RDA, 27), 296 (14), 295 (17), 281 (11), 280 (61) 279 (10), 278 (23), 268 (11) 267 (16).

$C_{19}H_{16}O_7$ (356.3)
Ber. : C 64.04 H 4.53
Gef. : C 63.73 H 4.62

## Beispiel 14

(+)-1-Desoxy-10-epi-$\alpha_1$-rhodomycinon (I, $R = CH_3$, $R_2 = OH$, $R_4 = OH$, $R_5 = H$)

Aus der polareren Zone der Chromatographie (s. oben) erhielt man 1.5 mg (1,4 %) der 10-epi-Verbindung ; Schmp. 120 °C. — IR : 3400 (OH), 1620 (Chinon cheliert), 1580 (C=C). — UV : $\lambda_{max}$(1gε) = 228 (3.89), 250 (4.11), 282 (3,64), 317 (3.23), 459 (3.44), 482 (3.49), 502 (3.39), 512 (3.34), 550 nm (2.78). — $^1$H-NMR : δ = 1.52 (s ; 3H, $CH_3$), 2.18 (dd, $J_{9a,9e}$ = 13.8, $J_{9a,10a}$ = 9.2 Hz ; 1H, 9a—H), 2.34 (ddd, $J_{9a,9e}$ = 13.8, $J_{9e,10a}$ = 7.2, $J_{9e,7e}$ = 1.2 Hz ; 1H, 9e—H), 3.68 (s ; 1H, OH), 3.96 (s ; 1H, OH), 416 (breites s ; 1H, OH), 4.77 (d, $J_{9e,7e}$ = 1.2 Hz ; 1H, 7e—H), 5.31 (dd, $J_{9a,10a}$ = 9.2, $J_{9e,10a}$ = 7.2 Hz ; 1H, 10a—H), 7.87 (mc ; 2H, 2—, 3—H), 8.37 (mc ; 2H, 1—, 4—H), 13.49, 13.82 (je s ; je 1H, je OH). — MS (195 °C) : m/e = 338 (M—$H_2O$, 5 %), 337 (13), 336 (61), 321 (19), 320 (M—$2H_2O$, 90 %), 305 (13), 304 (70), 303 (13), 298 (RDA, 5), 280 (11), 189 (13).

## Beispiel 15

(+)-1,7-Didesoxy-$\alpha_1$-rhodomycinon-7-yl-trifluoracetat (I, $R = CH_3$, $R_1 = H$, $R_2 = OH$, $R_5 = OH$)

Hergestellt nach der Vorschrift gem. Beispiel 13. Durch Ansäuern der Reaktionslösung nach 30 sec und anschließender schichtchromatographischer Auftrennung (Dichlormethan/Ether 96 : 4) erhält man folgendes Produktverhältnis mit zunehmer Polarität : Eingesetzte Menge Trifluoracetat : 100 mg (0.23 mmol) Ausbeute : Titelverbindung : 29 mg (28 %), Schmp. 220 °C ; Triol (I, $R = CH_3$, $R_2 = OH$, $R_5 = OH$) : 4 mg (4 %). Titelverbindung : IR : 3350 (OH, 1790) (C = 0, Trifluoracetat), 1630 (Chinon cheliert), 1585 (C=C). — $^1$N-NMR : δ = 1.43 (s ; 3H, $CH_3$), 2.19 (dd, $J_{gem}$ = 15.0, $J_{9a,10e}$ = 4.8 Hz ; 1H, 9a—H), 2.37 (ddd, $J_{gem}$ = 15.0, $J_{9e,10e}$ = 1.7, $J_{9e,7e}$ = 1.5 Hz ; 1H, 9e—H), 3.47 (breites Signal : 1H, OH),

3.95 (breites Signal : 1H, OH), 5.35 (dd, $J_{9a,10e}$ = 4.8, $J_{9e,10e}$ = 1.7 Hz ; 1H, 10e—H), 6.35 (d, $J_{9e,7e}$ = 1.5 Hz ; 1H, 7e—H), 7.87 (mc ; 2H, 2-3—H), 8.36 (mc ; 2H, 1—, 4—H), 13.21, 13.41 (je s ; je 1H, je OH). — MS (130 °C) : m/e = 452 ($M^+$, 27 %), 434 (11), 338 (43), 322 (13), 321 (27), 320 (100), 305 (23), 304 (60), 303 (11), 296 (14), 295 (25), 294 (16), 293 (12), 292 (27), 291 (12), 281 (30), 280 (63), 279 (16), 278 (43), 277 (33), 268 (13), 267 (19).

### Beispiel 16

(+)-1-Desoxy-β-rhodomycinon (I, R = $C_2H_5$, $R_1$ = H, $R_2$ = OH, $R_3$ = H, $R_4$ = H, $R_5$ = OH)

Wie in Beispiel 13 beschrieben wurden 36 mg (0.08 mmol) Trifluoracetat (III, R = $C_2H_5$, $R_1$ = H, $R_2$ = $OCOCF_3$, $R_3$ = H) bromiert und mit Natronlauge hydrolysiert. Nach schichtchromatographischer Trennung erhielt man aus der polaren Hauptfraktion 8 mg (30 %) Triol (Titelverbindung) mit Schmp. 212 °C. — $^1$H-NMR : δ = 1.13 (T, J = 7.4 Hz ; 3H, $CH_2CH_3$), 1.78 (dq, $J_{gem}$ = 15.0, J = 7.4 Hz ; 1H, $CH_2CH_3$), 1.91 (dq : 1H, $CH_2CH_3$), 2.15 (dd, $J_{gem}$ = 15.2, $J_{9a,10e}$ = 4.4 Hz ; 1H, 9a—H), 2.23 (ddd, $J_{gem}$ = 15.2, $J_{9e,10e}$ = 2.8, $J_{9e,7e}$ = 1.3 Hz ; 1H, 9e—H), 3.32 (s, austauschbar mit $CD_3OD$ ; 1H, 8—OH), 3.49 (d austauschbar ; $J_{10e,10-OH}$ = 3.6 Hz ; 1H, 10—OH), 3.65 (d austauschbar, $J_{7e,7-OH}$ = 1.2 Hz ; 1H, 7—OH), 4.91 (dd, $J_{7e,7-OH}$ = 1.2, $J_{9e,7e}$ = 1.3 Hz ; 1H, 7e—H), 5.23 (dd-d, $J_{10e,9a}$ = 4.4, $J_{10e,10-OH}$ = 3.6, $J_{10e,9e}$ = 2.8 Hz ; 1H, 10e—H), 7.87 (mc ; 2H, 2—, 3—H), 8.38 (mc ; 2H, 1—, 4—H), 13.46, 13.51 (je s ; je 1H, je OH). — MS (175 °C) : m/e = 371 (M + 1, 4 %), 370 ($M^+$, 19), 353 (18), 352 (77), 350 (14), 335 (19), 334 (65), 318 (22), 299 (18), 298 (RDA, 100), 297 (20), 296 (94), 295 (66), 281 (27), 280 (93), 278 (41), 277 (15), 270 (16), 269 (10), 268 (26), 267 (31), 252 (11).

$C_{20}H_{18}O_7$ (370.4)
Ber. : C 64.86 H 4.90
Gef. : C 64.62 H 4.99

### Beispiel 17

2-Formyl-6,11-dihydroxy-2-methyl-2H,3,4,7,12-tetrahydro-anthra [1,2-b]-pyran-7,12-dion (IV)

10 ml einer 1 proz. Natriumethanolat-Lösung wurden bei 20 °C mit 50 mg (II, R = $CH_3$, $R_1$ = OH) versetzt. Nach 45 min. Rühren unter Stickstoff wurde mit 50 ml Dichlormethan verdünnt, mit kalter 6N Salzsäure angesäuert und nach Zusatz von 100 ml Wasser extrahiert. Man wusch die organische Phase zweimal mit Wasser, trocknete über Natriumsulfat, filtrierte und dampfte das Lösungsmittel i.Vak. ab. Nach schichtchromatographischer Trennung (Dichlormethan/Ether 95 : 5) kristallisierten aus der polaren Fraktion 33 mg (80 %) mit Schmp. 228 °C (Zers.). — IR : 1725 (Aldehyd), 1610 (Chinon cheliert) $cm^{-1}$. — UV : $\lambda_{max}$(lgε) = 231 (4.59), 246 (4.20), 288 (3.93), 417 (3.64), 480 nm (4,04). — $^1$H-NMR : δ = 1.60 (s ; 3H, $CH_3$), 1.98 (dddd, $J_{gem}$ = 14.0, $J_{3a,4a}$ = 8.8, $J_{3a,4e}$ = 6.9, $J_{3a,CHO}$ = 1.0 Hz ; 1H, 3a—H), 2.34 (dt, $J_{gem}$ = 14.0, $J_{3e,4a,e}$ = 6.0 Hz ; 1H, 3e—H), 2.83 (dddd, $J_{gem}$ = 18.4, $J_{4a,3a}$ = 8,8, $J_{4a,3e}$ = 6.0, $J_{4a,5}$ = 1.2 Hz ; 1H, 4a—H), 2.90 (dddd, $J_{gem}$ = 18.4, $J_{4e,3a}$ = 6.9, $J_{4e,3e}$ = 6.0, $J_{4e,5}$ = 1.2 Hz ; 1H, 4e—H), 7.07 (t, $J_{4,5}$ = 1.2 Hz ; 1H, 5—H), 7.30 (dd, $J_{9,10}$ = 8.2, $J_{8,10}$ = 1.2 Hz ; 1H, 10—H), 7.63 (dd, $J_{9,10}$ = 8.2, $J_{8,9}$ = 7.6 Hz ; 1H, 9—H), 7.80 (dd, $J_{8,9}$ = 7.6, $J_{8,10}$ = 1.2 Hz ; 1H, 8—H), 9.76 (d, $J_{3a,CHO}$ = 1.0 Hz ; 1H, CHO), 13.11, 13.17 (je s ; je 1H, je OH).

$C_{19}H_{14}O_6$ (338.3)
Ber. : C 67.45 H 4.17
Gef. : C 67.34 H 4.11

### Beispiel 18

7-Desoxy-$\beta_1$-rhodomycinon (III, R = $CH_3$, $R_1$ = OH, $R_2$ = $R_3$ = H)

20 mg (0.06 mmol) (IV) wurden in 5 ml Methanol gelöst und unter Stickstoff mit 10 ml 1N Natronlauge versetzt. Bei Raumtemperatur tropfte man eine Natriumdithionit-Lösung bis zum Farbumschlag von blau nach gelborange zu und rührte über Nacht. Zur Reoxidation wurde an der Luft gerührt (30 min). Dann verdünnte man die Lösung mit 30 ml Wasser und säuerte mit kalter 6N Salzsäure bis zum Farbumschlag nach Rot an. Man extrahierte zweimal mit je 20 ml Dichlormethan, wusch mit Wasser, trocknete die Lösung über Natriumsulfat, filtrierte und dampfte das Lösungsmittel ab. Das Rohprodukt wurde schichtchromatographisch aufgetrennt (Dichlormethan/Ether 95 : 5). Aus der polaren Fraktion kristallisierten 8 mg (40 %) (IV) mit Schmp. 225 °C (Zers.). — IR : 3400 (OH), 1600 (Chinon cheliert), 1585 (C=C) $cm^{-1}$. — UV : $\lambda_{max}$(lgε) = 232 (4.35), 253 (4.36), 291 (3. 78), 433sh, 459 (3.91), 475 (3.95), 488 (4.03), 510 (3.86), 522 nm (3.89). — $^1$H-NMR : δ = 1.46 (s ; 3H, $CH_3$), 1.77 (dt, $J_{gem}$ = 13.8, J = 8.0 Hz ; 1H, 9a—H), 1.98 (d-dt, $J_{gem}$ = 13.8, J = 5.2, $J_{9e,7e}$ = 2.0 Hz ; 1H, 9e—H), 2.36 (breites Signal ; 1H, OH), 2.78 (dd, $J_{gem}$ = 18.6, $J_{7e,9e}$ = 2.0 Hz ; 1H, 7e—H), 2.95 (mc ; 2H, 10—$H_2$), 2.99 (d breit ; $J_{gem}$ = 18.6 Hz ; 1H, 7a—H), 7.29 (dd, $J_{2,3}$ = 8.4, $J_{2,4}$ = 1.2 Hz ; 1H, 2—H), 7.70 (t ; 1H, 3—H), 7.87 (dd, $J_{3,4}$ = 7.6, $J_{2,4}$ = 1.2 Hz ; 1H, 4—H), 12.30, 12.83, 13.65 (je s ; je 1H, je OH). — MS (220 °C) : m/e = 341 (M + 1, 21 %), 340 ($M^+$,

100), 323 (13), 322 (48), 307 (40), 298 (18), 297 (42), 283 (23), 282 (RDA, 81), 279 (18), 278 (12).

Veränderte man die oben angegebenen Versuchsbedingungen, so konnte bei Raumtemperatur bei einer eingesetzten Menge von 25 mg (IV) nach 2 h ausschließlich die Desoxy-Verbindung (Titelverbindung) erhalten werden (20 mg — 80 %). Wurde der Ansatz vor der Reduktion zur Leukoform auf 0 °C abgekühlt, so erhielt man bei einem Einsatz von 35 mg (IV) das trans-Diol (III, R = $CH_3$, $R_1$ = OH, $R_2$ = OH) (22 mg, 63 %) und (10 mg, 25 %) cis-Diol (III, R = $CH_3$, $R_1$ = OH, $R_3$ = OH).

### Beispiel 19

(+)-8c-n-Propyl-7,8,9,10-tetrahydro-1,6,7r,8t,11-pentahydroxy-5,12-naphthacenchinon = 13-Homo-γ-rhodomycinon (III, R = n-$C_3H_7$, $R_1$ = OH, $R_2$ = OH, $R_3$ = H)

Eine Lösung von 100 mg (0.23 mmol) (II, R = n-$C_3H_7$) in 30 ml Dichlormethan wurde mit 30 ml einer 0.2 N NaOH-Lösung versetzt. Nach Zugabe einer Spatelspitze Tetra butylammoniumhydrogensulfat rührte man bis zur vollständigen Umsetzung des Ausgangsmaterials (II, R = n-$C_3H_7$, $R_1$ = OH) (ca. 1 h ; DC-Kontrolle). Die Mischung wurde mit Eis gekühlt und unter Stickstoff mit einer Lösung von 100 mg Natriumdithionit in 5 ml Wasser reduziert. Nach 30 min Rühren unter Eiskühlung leitete man zur Reoxidation Luft durch die Lösung (ca. 20 min), säuerte mit verd. Salzsäure an und extrahierte zweimal mit je 30 ml Dichlormethan. Die organische Phase wurde zweimal mit Wasser gewaschen, über Natriumsulfat getrocknet, filtriert und eingeengt. Der Rückstand wurde schichtchromatographisch aufgetrennt (Dichlormethan/Ether 90 : 10). Aus der unpolaren Fraktion der Chromatographie kristallisierten 57 mg (65 %) trans-Diol (Titelverb.) mit Schm. 219 °C (Zers.). — IR : 3450 (OH), 2945-2850 (CH), 1600 (Chinon cheliert), 1590 (C=C) $cm^{-1}$. — UV : $\lambda_{max}$(1gε) = 220sh, 235 (4.41), 252 (4.34), 290 (3.77), 435sh, 466 (3.94) 479 (3.98), 491 (4.06), 510 (3.91), 525 nm (3.92). — MS (290 °C) : m/e = 384 ($M^+$, 44 %), 368 (24), 366 (M — $H_2O$, 16), 350 (13), 349 (12), 348 (M — $2H_2O$, 40), 337 (12), 319 (16), 307 (11), 298 (RDA, 44), 297 (18), 296 (27), 295 (50), 285 (10), 284 (14), 283 (13), 282 (18), 271 (16), 270 (87), 269 (12), 255 (11).

$C_{21}H_{20}O_7$ (384.4)
Ber. : C 65.62 H 5.24
Gef. : C 65.41 H 5.29

### Beispiel 20

(+)-8t-n-Propyl-7,8,9,10-tetrahydro-1,6,7r,8c,11-pentahydroxy-5,12-naphthacenchinon (III, R = n-$C_3H_7$, $R_1$ = OH, $R_2$ = H, $R_3$ = OH)

Aus der polaren Fraktion der Chromatographie (s. oben) kristallisierten 20 mg (23 %) cis-Diol (Titelverbindung) mit Schmp. 169 °C. — IR : 3360 (OH), 2950-2850 (CH), 1600 (Chinon cheliert, C=C) $cm^{-1}$. — UV : $\lambda_{max}$(1gε) = 218sh, 233 (4.43), 251 (4.31), 291 (3.78), 464 (3.94), 477 (3.99), 491 (4.06), 511 (3.92), 525 nm (3.90). — $^1$H-NMR (400 MHz) : δ = 0.95 (t, J = 7.0 Hz ; 3H, $CH_3$), 1.54 (mc ; 4H, $CH_2CH_2CH_3$), 1.76 (ddd, $J_{gem}$ = 13.5, $J_{9a,10a}$ = 12.9, $J_{9a,10e}$ = 6.3 Hz ; 1H, 9a—H), 2.09 (ddd, $J_{gem}$ = 13.5, $J_{9e,10a}$ = 7.2 Hz, $J_{9e,10e}$ = 5.8 Hz ; 1H, 9e—H), 2.74 (dt, $J_{gem}$ = 19.4, J = 6.6 Hz ; 1H, 10—H), 2.79 (s ; 1H, 8—OH), 3.02 (dt, $J_{gem}$ = 19.4, J = 6.2 Hz ; 1H, 10—H), 3.75 (d, J E 2.0 Hz ; 1H, 1—OH), 4.80 (s breit ; 1H, 7a—H), 7.32 (dd, $J_{2,3}$ = 8.4, $J_{4,2}$ = 1.1 Hz ; 1H, 2—H), 7.71 (t ; 1H, 3—H), 7.89 (dd, $J_{3,4}$ = 7.4, $J_{2,4}$ = 1.1 Hz ; 1H, 4—H), 12.25, 12.75, 14.07 (je s ; je 1H, je OH). — MS (240 °C) : m/e = 385 (M + 1, 18 %), $384^+$, 76), 366 (27), 348 (14), 337 (11), 298 (RDA, 52), 296 (34), 295 (56), 285 (11), 271 (18), 270 (100), 203 (13).

### Beispiel 21

(+)-γ-Rhodomycinon (III, R = $C_2H_5$, $R_1$ = OH, $R_2$ = OH) und (+)-7-epi-γ-Rhodomycinon (III, R = $C_2H_5$, $R_1$ = OH, $R_3$ = OH)

Analog zu der in Beispiel 19 beschriebenen Vorschrift erhielt man aus 425 mg (1 mmol) Dichlorid (II, R = $C_2H_5$, $R_1$ = OH) 218 mg (59 %) trans-Diol und 81 mg (22 %) cis-Diol, die mit früher hergestellten Referenzproben identisch waren.

### Beispiel 22

(+)-7-Desoxy-13-homo-γ-rhodomycinon-7-yl-trifluoracetat (III, R = n-$C_3H_7$, $R_1$ = OH, $R_2$ = $OCOCF_3$)

Wie in Beispiel 12 beschrieben wurden 50 mg (0.13 mmol) trans-Diol (III, R = n-$C_3H_7$, $R_1$ = OH, $R_2$ = OH) zum Monotrifluoracetat (III, $R_2$ = $OCOCF_3$) umgesetzt. Ausbeute : 62 mg (quantitativ) ; Schmp. 171 °C. — IR : 3475 (OH), 2950-2840 (CH), 1790 (C=O, Trifluoracetat), 1680 (Chinon), 1600 (Chinon cheliert) $cm^{-1}$. — UV : $\lambda_{max}$(1gε) = 222sh, 233 (4.43), 252 (4.33), 291 (3.84), 464 (3.95), 479 (4.00), 490 (4.06), 509 (3.93), 524 nm (3.91). — $^1$H-NMR : δ = 1.00 (dt, J = 6.6, J = 1.8 Hz ; 3H, $CH_3$), 1.59 (mc ; 4H,

$CH_2CH_2CH_3$), 1.86 (ddd, $J_{gem}$ = 13.9, $J_{9a,10a}$ = 11.6, $J_{9a,10e}$ = 6.0 Hz; 1H, 9a—H), 2.11 (mc; 1H, 9e—H), 2.88 (ddd, $J_{gem}$ = 19.3, $J_{9a,10a}$ = 11.6, $J_{10a,9e}$ = 6.0 Hz; 1H, 10a—H), 3.09 (mc; 1H, 10e—H), 6.24 (d, $J_{9e,7e}$ = 1.2 Hz; 1H, 7e—H), 7.31 (dd, $J_{2,3}$ = 8.4, $J_{2,4}$ = 1.0 Hz; 1H, 2—H), 7.71 (t; 1H, 3—H), 7.87 (dd, $J_{3,4}$ = 7.6, $J_{2,4}$ = 1.0 Hz; 1H, 4—H), 12.15, 12.67, 13.56 (je s; je 1H, je OH). — MS (250 °C): m/e = 480 ($M^+$, 12 %), 366 (29), 348 (12), 339 (20), 338 (94), 323 (15), 309 (20), 297 (15), 296 (14), 295 (24), 270 (19), 203 (13).

Beispiel 23

13-Homo-β-rhodomycinon (I, R = n-$C_3H_7$, $R_1$ = OH, $R_2$ = OH, $R_5$ = OH)

Nach dem für Beispiel 13 beschriebenen Verfahren wurden 30 mg (0.06 mmol) Trifluoracetat gemäß Beispiel 20 zu 8 mg (30 %) Titelverbindung mit Schmp. 218 °C (Zers.) hydroxyliert. — $^1$H-NMR: δ = 1.03 (t, J = 7.2 Hz; 3H, $CH_3$), 1.48-1.84 (m; 4H, $CH_2CH_2CH_3$), 2.16 (dd, $J_{gem}$ = 15.0, $J_{9a,10e}$ = 4.0 Hz; 1H, 9a—H), 2.23 (ddd, $J_{gem}$ = 15.0, $J_{9e,10e}$ = 1,6, $J_{9e,7e}$ = 1.0 Hz; 1H, 9e—H), 2.67 (breites Signal; 1H, OH), 3.31 (s breit; 1H, OH), 3.66 (s; 1H, OH), 4.86 (d, $J_{9e,7e}$ = 1.0 Hz; 1H, 7e—H), 5.22 (mc; γ/2 = 9.2 Hz, 10e—H), 7.34 (dd, $J_{2,3}$ = 8.6, $J_{2,4}$ = 1.0 Hz; 1H, 2—H), 7.73 (t; 1H, 3—H), 7.90 (dd, $J_{3,4}$ = 7.2, $J_{2,4}$ = 1.0 Hz; 1H, 4—H), 12.12, 12.87, 13.58 (je s; je 1H, je OH). — MS (200 °C): m/e = 400 ($M^+$, 9 %), 384 (19), 383 (12), 382 (48), 380 (27), 366 (11), 365 (13), 364 (45), 349 (13), 348 (46), 335 (18), 319 (18), 315 (14), 314 (RDA, 78), 313 (18), 312 (82), 311 (55), 298 (19), 297 (22), 296 (77), 295 (25), 294 (31), 293 (12), 286 (15), 285 (11), 284 (18), 283 (21), 270 (32).

$C_{21}H_{20}O_8$ (400.4)
Ber.: C 63.00 H 5.03
Gef.: C 62.89 H 5.12

Beispiel 24

13-Homo-α-rhodomycinon (I, R = n-$C_3H_7$, $R_1$ = OH, $R_2$ = OH, $R_4$ = OH)

Man ließ eine Lösung von 6 mg 13-Homo-β-rhodomycinon (Beispiel 21) in 1 ml Trifluoressigsäure 24 h bei Raumtemperatur stehen, versetzte unter Rühren mit 20 ml 1N NaOH, säuerte nach einer Minute mit verd. Salzsäure an und extrahierte mit Dichlormethan. Das Lösungsmittel wurde i. Vak. abgedampft und der Rückstand schichtchromatographisch getrennt (Dichlormethan/Methanol 98 : 2). Aus der weniger polaren Zone wurden 3 mg des Ausgangsmaterials zurückgewonnen, aus der polaren Zone erhielt man 0.5 mg Triol (Titelverbindung) mit Schmp. 207 °C (Zers.). — $^1$H-NMR: δ = 1.00 (mc; 3H, $CH_3$), 1.40-1.70 (m; 4H, $CH_2CH_2CH_3$), 2.30 (mc; 2H, 9a, e—H), 4.77 (mc; 1H, 7—H), 5.44 (mc; 1H, 10—H), 7.34 (d breit; $J_{2,3}$ = 8.5 Hz: 1H, 2—H), 7.73 (t; 1H, 3—H), 7.90 (d breit; $J_{3,4}$ = 7.6 Hz; 1H, 4—H), 12.09, 13.16, 13.60 (je s; je 1H, je OH). — MS (175 °C): m/e = 400 ($M^+$, 6 %), 384 (15), 382 (31), 380 (27), 366 (12), 365 (11), 364 (39), 351 (11), 349 (17), 348 (64), 335 (15), 323 (11), 319 (30), 314 (49), 313 (11), 312 (44), 311 (30), 298 (15), 297 (18), 296 (67), 295 (23), 294 (23), 293 (10), 291 (11), 286 (15), 284 (13), 283 (15), 270 (40).

**Patentansprüche**

1. Verfahren zur Herstellung von Verbindungen der Formel I

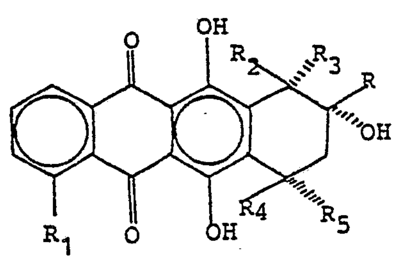

(I)

worin R $C_1$-$C_4$-Alkyl, $R_1$ Wasserstoff oder Hydroxy bedeuten, $R_2$ und $R_3$ voneinander verschieden sind und jeweils Wasserstoff oder Hydroxy bedeuten sowie $R_4$ und $R_5$ ebenfalls voneinander verschieden sind und Wasserstoff oder Hydroxy bedeuten, dadurch gekennzeichnet, daß man eine Verbindung der Formel

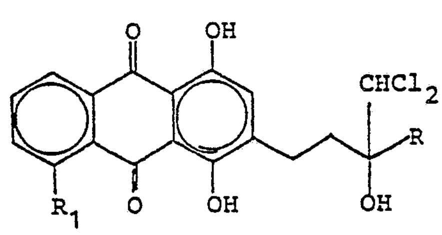

(II)

worin R und $R_1$ die oben genannte Bedeutung haben, entweder

a) falls $R_1$ Wasserstoff bedeutet, mit einer Alkalilauge unter Zusatz eines Reduktionsmittels, vorzugsweise Natriumdithionit, bei 0 °C umsetzt und das erhaltene Produkt anschließend mit Luft oxidiert und ansäuert, wobei man eine Verbindung der Formel III

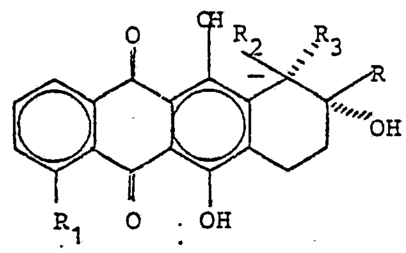

(III)

erhält, worin $R_1$ Wasserstoff bedeutet und R, $R_2$ und $R_3$ die oben angegebene Bedeutung haben, oder

b) falls $R_1$ die OH-Gruppe bedeutet, in einer Phasentransfer-Reaktion mit einer Alkalilauge in einen chlorierten Kohlenwasserstoff, unter Zusatz von Tetrabutylammoniumhydrogensulfat und anschließende Reduktion mit einem geeigneten Reduktionsmittel, bei 0 °C umsetzt zu einer Verbindung der Formel III, worin $R_1$ Hydroxy bedeutet und R, $R_2$ und $R_3$ die oben angegebene Bedeutung haben, oder

c) falls $R_1$ die Hydroxygruppe und R $CH_3$ bedeutet, mit Alkalimethanolat bei Raumtemperatur unter Inertgas umsetzt zu einer Verbindung der Formel IV

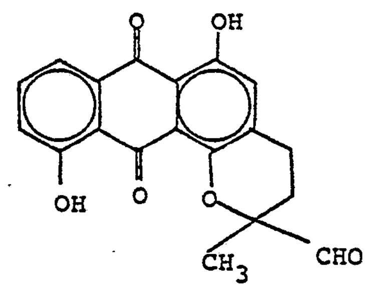

(IV)

und den erhaltenen cyclischen Aldehyd durch Umsetzen mit einem geeigneten Reduktionsmittel, vorzugsweise Natriumdithionit bei 0 °C und anschließende Luftoxidation und Ansäuern überführt in eine Verbindung der Formel III, worin R $CH_3$ und $R_1$ OH bedeutet, und $R_2$ und $R_3$ die oben angegebene Bedeutung haben, und

d) eine Verbindung der Formel III, worin R, $R_1$ und $R_3$ die oben genannte Bedeutung haben und $R_2$ die OH-Gruppe bedeutet, durch Veresterung mit Hexafluoressigsäureanhydrid in eine Verbindung der Formel III, worin $R_2$ den Rest $OCOCF_3$ darstellt, überführt, und

e) eine Verbindung der Formel III, worin R und $R_1$ die oben angegebene Bedeutung haben, $R_3$ Wasserstoff und $R_2$ den Rest $-OCOCF_3$ darstellt, nach üblichen Methoden bromiert und anschließend hydrolysiert zu einer Verbindung der Formel I.

2. Verfahren zur Herstellung von Verbindungen der Formel I gemäß Anspruch 1, dadurch gekennzeichnet, daß man eine Verbindung der Formel II

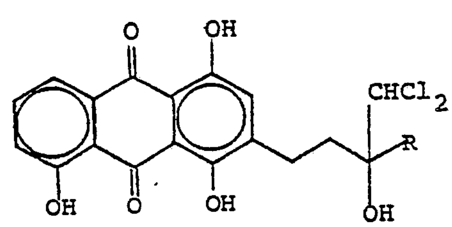

(II)

worin R $C_1$-$C_4$-Alkyl bedeutet, in einer Phasentransfer-Reaktion mit einer Alkalilauge in Dichlormethan unter Zusatz von Tetrabutylammoniumhydrogensulfat und anschließende Reduktion mit Natriumdithionit bei 0 °C umsetzt zu einer Verbindung der Formel III

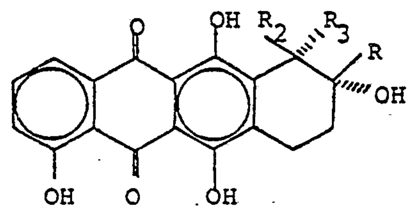

(III)

worin R, $R_2$ und $R_3$ die in Anspruch 1 genannten Bedeutungen haben, die erhaltene Verbindung der Formel III, worin $R_2$ eine OH-Gruppe bedeutet, mit Hexafluoressigsäureanhydrid in einer Verbindung der Formel III überführt, worin $R_2$ den Rest $—OCOCF_3$ darstellt und die erhaltene Verbindung nach üblichen Methoden bromiert und anschließend hydrolysiert.

3. Verbindungen der Formel II

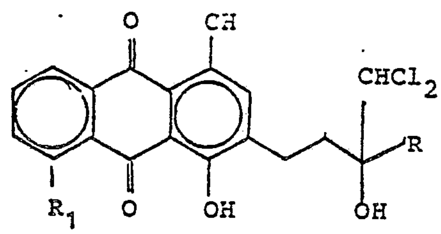

worin R $C_1$-$C_4$-Alkyl und $R_1$ Wasserstoff oder Hydroxy darstellt.

**Claims**

1. A process for the preparation of a compound of the formula I

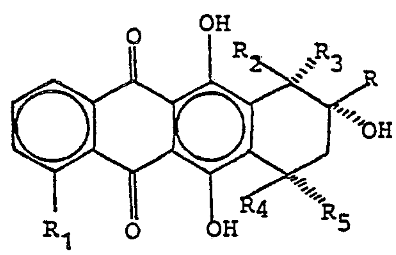

(I)

in which R denotes $C_1$-$C_4$-alkyl, $R_1$ denotes hydrogen or hydroxyl, and $R_2$ and $R_3$ differ from one another and each denote hydrogen or hydroxyl, and $R_4$ and $R_5$ likewise differ from one another and denote hydrogen or hydroxyl, comprises taking a compound of the formula

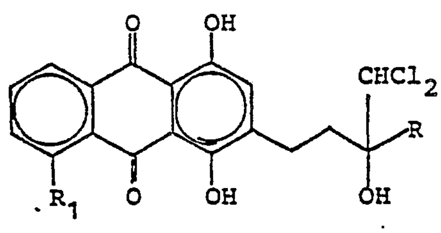

(II)

in which R and $R_1$ have the abovemention meaning, and either

a) where $R_1$ denotes hydrogen, reacting it with an alkali metal hydroxide solution with the addition of a reducing agent, preferably sodium dithionite, at 0 °C and then oxidizing the resulting product with air and acidifying, whereupon a compound of the formula III

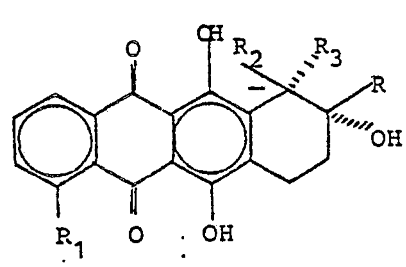

(III)

in which $R_1$ denotes hydrogen and R, $R_2$ and $R_3$ have the abovementioned meaning, is obtained or

b) where $R_1$ denotes the OH group, reacting it in a phase-transfer reaction with an alkali metal hydroxide solution in a chlorinated hydrocarbon, preferably dichloromethane, with the addition of

tetrabutylammonium hydrogen sulfate, followed by reduction with a suitable reducing agent, preferably sodium dithionite, at 0 °C to give a compound of the formula III, in which $R_1$ denotes the OH group and $R_1$, $R_2$ and $R_3$ have the abovementioned meaning, or

c) where $R_1$ denotes the OH group and R denotes $CH_3$, reacting it with an alkali metal methanolate at room temperature under an inert gas to give a compound of the formula IV

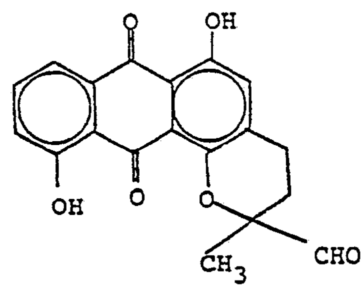

(IV)

and converting the resulting cyclic aldehyde, by reaction with a suitable reducing agent, preferably sodium dithionite, at 0 °C followed by oxidation with air and acidification, into a compound of the formula III, in which R denotes $CH_3$ $R_1$ denotes the OH group and $R_2$ and $R_3$ have the abovementioned meaning, and

d) converting a compound of the formula III, in which R, $R_1$ and $R_3$ have the abovementioned meaning, and $R_2$ denotes the OH group by esterification with hexafluoroacidic anhydride into a compound of the formula III, in which $R_2$ represents the $-OCOCF_3$ radical, and

e) brominating a compound of the formula III, in which R and $R_1$ have the abovementioned meaning, $R_3$ represents hydrogen and $R_2$ represents the $-OCOCF_3$ radical, by customary methods and then carrying out hydrolysis to give a compound of the formula I.

2. The process for the preparation of a compound of the formula I as claimed in claim 1, wherein a compound of the formula II

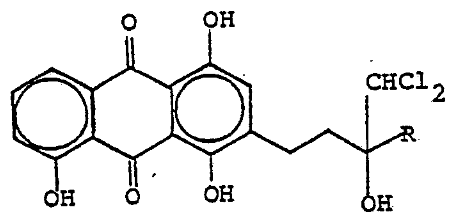

(II)

in which R denotes $C_1$-$C_4$-alkyl, is reacted in a phase-transfer reaction with an alkali metal hydroxide solution in dichloromethane, with the addition of tetrabutylammonium hydrogen sulfate, followed by reduction with sodium dithionite at 0 °C to give a compound of the formula III

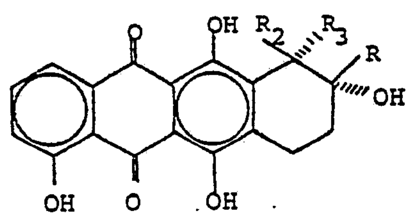

(III)

in which R, $R_2$ and $R_3$ have the meanings indicated in claim 1, the resulting compound of the formula III, in which $R_2$ denotes an OH group, is converted with hexafluoroacetic anhydride into a compound of the formula III, in which $R_2$ represents the $-OCOCF_3$ radical, and the resulting compound is brominated by customary methods and then hydrolyzed.

3. A compound of the formula II

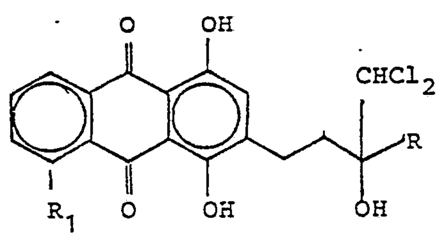

(II)

in which R represents $C_1$-$C_4$-alkyl and $R_1$ represents hydrogen or hydroxyl.

**Revendications**

1. Procédé de préparation de composés de formule

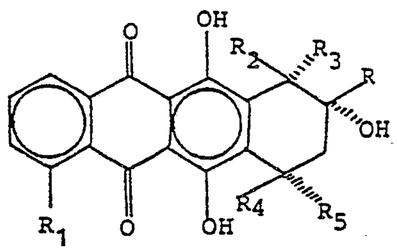

(I)

dans laquelle R représente un groupe alkyle en $C_1$-$C_4$, $R_1$ représente l'hydrogène ou un groupe hydroxy, $R_2$ et $R_3$ sont différents l'un de l'autre et représente l'hydrogène ou un groupe hydroxy et $R_4$ et $R_5$ sont également différents l'un de l'autre et représente l'hydrogène ou un groupe hydroxy, caractérisé en ce que l'on fait réagir un composé de formule

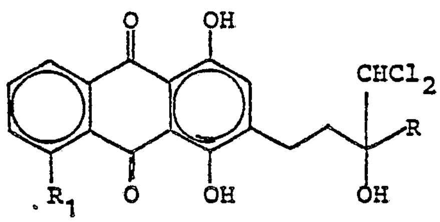

(II)

dans laquelle $R_1$ et R ont les significations indiquées ci-dessus, ou bien

a) au cas où $R_1$ représente l'hydrogène, avec une solution d'alcali en ajoutant un réducteur, de préférence du dithionite de sodium, à 0 °C et ensuite on oxyde le produit obtenu à l'air et on l'acidifie, ce qui donne un composé de formule III

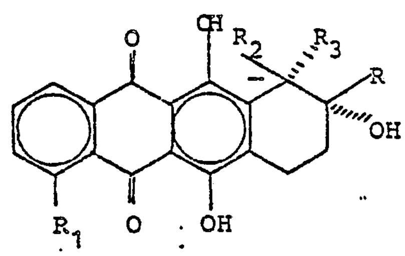

(III)

dans laquelle $R_1$ représente l'hydrogène et R, $R_2$ et $R_3$ ont la signification indiquée ci-dessus, ou bien

b) au cas où $R_1$ représente le groupe OH, dans une réaction de transfert de phases, avec une solution d'alcali dans un hydrocarbure chloré, de préférence le dichlorométhane, avec addition d'hydrogénosulfate de tétrabutylammonium et ensuite réduction avec un réducteur approprié, à 0 °C, pour obtenir un composé de formule III, dans laquelle $R_1$ représente le groupe OH et R, $R_2$ et $R_3$ ont la signification indiquée ci-dessus, ou bien

c) au cas où $R_1$ représente le groupe OH et R représente $CH_3$, on le fait réagir avec un méthanolate alcalin à la température ambiante sous gaz inerte, pour obtenir un composé de formule IV

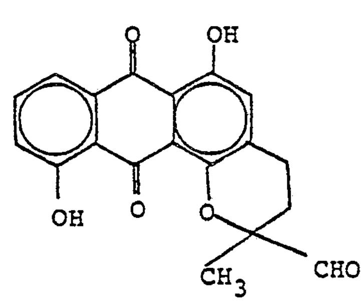

(IV)

et on transforme d'aldéhyde cyclique obtenu, par réaction avec un réducteur approprié, de préférence le

dithionite de sodium à 0 °C et ensuite oxydation à l'air et acidification, en un composé de formule III dans laquelle R représente $CH_3$ et $R_2$ et $R_3$ ont la signification indiquée ci-dessus, et

d) on transforme un composé de formule III, dans laquelle R, $R_1$ et $R_3$ ont la signification indiquée ci-dessus et $R_2$ représente le groupe OH, par estérification avec de l'anhydride hexafluoroacétique, en un composé de formule III, dans laquelle $R_2$ représente le radical $—OCOCF_3$, et

e) on brome par les méthodes habituelles un composé de formule III, dans laquelle R et $R_1$ ont la signification indiquée ci-dessus, $R_3$ représente l'hydrogène et $R_2$ représente le radical $—OCOCF_3$, et ensuite on l'hydrolyse en un composé de formule I.

2. Procédé de préparation de composé de formule I selon la revendication 1, caractérisé en ce que l'on fait réagir un composé de formule II

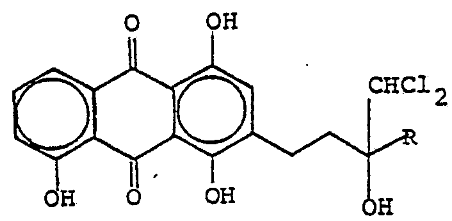

(II)

dans laquelle R représente un groupe alkyle en $C_1$-$C_4$, dans une réaction de transfert de phases, avec une solution d'alcali dans le dichlorométhane avec addition d'hydrogénosulfate de tétrabutylammonium et ensuite réduction par le dithionite de sodium à 0 °C pour obtenir un composé de formule III

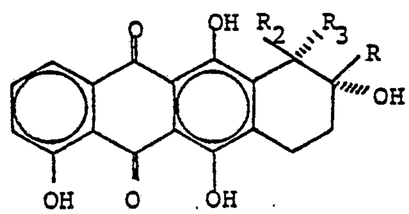

(III)

dans laquelle R, $R_2$ et $R_3$ ont les significations indiquées dans la revendication 1, on transforme le composé obtenu de formule III dans laquelle $R_2$ représente un groupe OH, avec de l'anhydride hexafluoroacétique en un composé de formule III dans laquelle $R_2$ représente le radical $—OCOCF_3$ et on brome le composé obtenu par les méthodes habituelles et ensuite on l'hydrolyse.

3. Composé de formule II

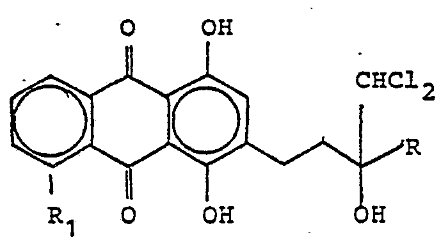

dans laquelle R représente un groupe alkyle en $C_1$-$C_4$ et $R_1$ représente l'hydrogène ou un groupe hydroxy.